# EUROPEAN PATENT SPECIFICATION

(11) **EP 3 278 798 B1**
(45) Date of publication and mention of the grant of the patent: **06.02.2019**
(21) Application number: 16382378.4
(22) Date of filing: 01.08.2016
(51) Int. Cl.: A61K 31/192, A61K 35/644, A23L 21/20, A23L 33/10, A61P 31/10

(54) **SYNERGISTIC COMPOSITION COMPRISING PROPOLIS AND CARNOSIC ACID FOR USE THEREOF IN TREATING AND PREVENTING INFECTIONS CAUSED BY SPECIES OF THE CRYPTOCOCCUS NEOFORMANS FUNGUS**
SYNERGISTISCHE ZUSAMMENSETZUNG MIT PROPOLIS UND CARNOSINSÄURE ZUR VERWENDUNG BEI DER BEHANDLUNG UND VORBEUGUNG VON INFEKTIONEN DURCH SPEZIES DES CRYPTOCOCCUS NEOFORMANS-PILZES
COMPOSITION SYNERGIQUE COMPRENANT DE LA PROPOLIS ET ACIDE CARNOSIQUE POUR LEUR UTILISATION DANS LE TRAITEMENT ET LA PRÉVENTION D'INFECTIONS PROVOQUÉES PAR DES ESPÈCES DE CHAMPIGNONS CRYPTOCOCCUS NEOFORMANS

(43) Date of publication of application: 07.02.2018
(73) Proprietor: VITALGAIA ESPAÑA, S.L., 30005 Murcia (ES)
(72) Inventor: Lozano Teruel, José Antonio, 30005 MURCIA (ES); Argüelles Ordóñez, Juan Carlos, 30005 MURCIA (ES); Argüelles Prieto, Alejandra, 30005 MURCIA (ES); Sánchez-Fresneda Pinto, Ruth, 30005 MURCIA (ES); Guirao Abad, José Pedro, 30005 MURCIA (ES); Alburquerque Gonzalez, Begoña, 30005 MURCIA (ES)
(74) Representative: Elzaburu S.L.P.

(56) References cited:
- Fabrício Freitas Fernandes ET AL: "The "in vitro" antifungal activity evaluation of propolis G12 ethanol extract on Cryptococcus neoformans", Revista do Instituto de Medicina Tropical de São Paulo, 1 April 2007 (2007-04-01), pages 93-95, XP055334213, Brazil DOI: 10.1590/S0036-46652007000200005 Retrieved from the Internet: URL:http://www.scielo.br/pdf/rimtsp/v49n2/ 05.pdf
- Ademar A. Da Silva Filho ET AL: "Antimicrobial Activity of the Extract and Isolated Compounds from Baccharis dracunculifolia D. C. (Asteraceae)", Zeitschrift für Naturforschung C, 1 February 2008 (2008-02-01), pages 40-46, XP055334325, Germany DOI: 10.1515/znc-2008-1-208 Retrieved from the Internet: URL:http://www.znaturforsch.com/s63c/s63c0 040.pdf
- MARA BELN AGERO ET AL: "Argentinean Andean propolis associated with the medicinal plantCav. (Zygophyllaceae). HPLCMS and GCMS characterization and antifungal activity", FOOD AND CHEMICAL TOXICOLOGY, PERGAMON, GB, vol. 49, no. 9, 6 May 2011 (2011-05-06), pages 1970-1978, XP028266592, ISSN: 0278-6915, DOI: 10.1016/J.FCT.2011.05.008 [retrieved on 2011-05-12]
- MARIANO PERTINO ET AL: "Synthesis, Antiproliferative and Antifungal Activities of 1,2,3-Triazole-Substituted Carnosic Acid and Carnosol Derivatives", MOLECULES, vol. 20, no. 5, 14 May 2015 (2015-05-14), pages 8666-8686, XP055334206, DOI: 10.3390/molecules20058666

## Description

### Field of the invention

The present invention refers to prevention and treatment of meningitis, pneumonia and other infections caused by species of the opportunistic fungus *Cryptococcus Neoformans,* in humans and animals. In particular, the present invention refers to a synergistic composition comprising propolis and carnosic acid for use in the above therapeutic application, as set forth in the claims.

### BACKGROUND OF THE INVENTION

The applicant has tested antimicrobial formulations with other substances, for example, bromelaine, Epigallocatechin gallate or Melaleuca alternifolia. These formulations did not show satisfactory results.

Sinergy means, by definition, according to the text adapted from the English original written by Barb Reutenbach, that the combined effect of the interaction of the parts creates a greater effect than the individual sum thereof. In fact, in the same sense as that set forth in the concept that has just been shown, it can be seen that the essence of the "general system theory (GST)", initially set out by Bertalanffy and taken to new and higher levels, as by Ervin Laszlo, is that synergies can be generated in a living system, but the effect of these interactions exceeds that generated by the sum of the action of the isolated parts.

Synergy allows obtaining a result which is better than that of the simple sum of the parts. The term comes from the Greek word "sin" meaning "with" and "ergos" meaning "work". Synergy also includes ways of collaboration which touch the deepest part of human nature, as creative thinking and conscious purpose.

Peter Andrew Corning Director of the Institute for the Study of Complex Systems in Washington, in the assay "The Synergism hypothesis: On the Concept of Synergy and its Role in the Evolution of Complex Systems", went deeper in a line introduced by Bertalanffy, with his hypothesis of synergy as a creativity source in biological evolution, and has played a central role in the development of organizational complexity in nature; so synergy is a phenomenon that involves creativity and complexity.

By transferring this synergistic concept and the accompanying assertions to the world of the fungal infections, mycosis is found to have increased dramatically in the last two decades, associated to the increase of the range of population undergoing situations of immunosuppression. Said infections are characterized by the length and persistence thereof in the human body. On the other hand, the therapeutic treatment therefor is complicated, since fungi and human beings share the same cell organization pattern (eukaryotic organisms), which hinders a high selective toxicity of the antifungal agents, as well as the resistance that has been reported in the clinical practice. Therefore, the maximum difficulty that science has faced regarding mycosis, as the current state of the art reveals, is healing time, this fact becoming the most difficult obstacle to overcome for eradication thereof, since whereas in the area of bacteria being treated with antibiotics the therapeutic approach implies a short time, the scenario becomes complicated when dealing with a fungal infection or opportunistic yeasts, such as Candida, capable of cohabiting in the host body for months or years, and which can lead to fatal processes if they cause generalized candidemia or septicaemia.

In view of such a complex problem, there is no doubt that the most valuable target in the fight against fungi is to find a synergistically acting mechanism which, in the shortest time possible is able to fight infections by Candida sp. and other pathogenic fungi, thereby this would deserve to be qualified as the highest inventive step in the field of fighting against fungal infections. In this regard, the carnosic acid-propolis synergy claimed in this patent application from Vitalgaia, is unique in the current state of the art since it achieves a marked fungal action in an hour, thus scientifically demonstrating that it is not a mere sum or addition of the effects of two individually isolated compounds, but rather that synergy has been mathematically proved, scientifically, through the corresponding isobologram.

Pathologies caused by opportunistic fungi have drastically increased in the las decades. Data from the WHO estimate an incidence of two millions of new cases per year, showing a mortality rate of about 20-40%, depending on the scenario. The section of immunocompromised population is the most susceptible one towards septicemic mycosis, including HIV patients or those undergoing intense anti-tumour and antibiotic therapies, as well as those being treated in intensive care units. Several circumstances may explain this aggravation: the limited availability of safe and efficient antifungals; enhanced resistance to conventional compounds, and growing isolation of new strains responsible of nosocomial outbreak, such as the "non-albicans" species of the genus Candida, Aspergillus and Cryptococcus. The latter fungus will be an object of the present patent.

### General biology of Cryptococcus

It is an encapsulated yeast which belongs to the class Basidiomycetes, as well as the etiologic agent of cryptococcal meningitis, an opportunistic mycosis being potentially severe in immunosuppressed patients, mainly those suffering from HIV (AIDS), although it can also affect immunocompetent individuals (Crowe et al., 1991; Currie and Casadevall, 1994). It includes two species: C. neoformans, a microorganism universally distributed which is found, above all, in natural habitats originating in pigeon guano, droppings from different birds and bats, and mires (Casadevall and Perfect, 1998). In contrast, Cryptococcus gattii, frequently affects healthy people causing pneumonia in the majority of the cases. It is generally isolated from subtropical or tropical areas, although it has been collected in milder areas, having increased in the last years in some areas of the North Pacific. Both show a significant prevalence in developing countries.

### Clinical importance

The most severe pathology is the so called cryptococcal meningitis (Meningoencephalitis) or cryptococcosis (Casadevall and Perfect, 1998). Contagium begins by inhalation of blastoconidia (yeast forms) or spores of Cryptococcus neoformans and C. gattii, being the lung the first colonized organ. Certain serotypes feature a higher degree of virulence (Casadevall and Perfect 1998, Velagapudi et al 2009). The evolution of the disease depends on the host immune response, being a controlled response in the case of healthy individuals, except in case of complications. However, yeasts can replicate within macrophages, the latter acting as a reservoir wherein they can stay latent for years. Under immunosuppression conditions (mainly with a decrease in the number of T cells) a massive colonization is produced, which involves dissemination from the lungs through the blood stream and accommodation thereof in the central nervous system, wherein it causes meningitis and meningoencephalitis, with fatal results if it is not treated appropriately.

The main virulence factors of Cryptococcus are an outer polysaccharide capsule having anti-phagocytic activity and the capacity to gather melanin. It also features other properties, such as urea and phospholipases production conferring some advantage for survival thereof inside the host. Although morphogenesis is a characteristic which is common to many pathogenic fungi, Cryptococcus does not form hyphae or pseudo-hyphae, even though during colonization in vivo it experiments relevant morphological changes in cell morphology. The most important ones are: A) Widening of the outer capsule; and B) Overall cell growth, resulting in "giant" or "titan" cell formation. The molecular mechanisms involved in these processes regulation still remain unknown.

Infections produced by C. neoformans have been an important cause of morbidity and mortality in HIV patients, since between 5-10% of individuals undergoing CD4+ lymphopenia developed cryptococcosis at the end of the XX century (Casadevall and Perfect, 1998). Although incidence of cryptococcosis has reduced significantly in developed countries thanks to the introduction of High Activity Antiretroviral Therapy (HAART), the mortality rate associated thereto continues being very high (Chottanapund et al., 2007; Dromer et al., 2007; Friedman et al., 2005; Jongwutiwes et al., 2007; Lortholary et al., 2006; Mirza et al., 2003). In developing areas, incidence of cryptococcosis is far from being controlled, with an estimation of 720,000 new cases per year in Sub-Saharan Africa and 120,000 in South-East Asia (Park et al., 2009). In these areas, together with South-America and the Caribbean, the mortality rate ranges between 55-70%, with a number of deaths per year that amounts to more than 650,000 people. Cryptococcus gattii has traditionally been associated to the eucalyptus bark (Fortes et al., 2001) and it seems to have a global distribution which is different to that of C. neoformans, being located in tropical and sub-tropical areas, but recent studies indicate that C. gattii could have a wider distribution (Campbell et al., 2005; Chen et al., 2008). Similarly to C. neoformans, C. gattii is transmitted by inhalation. However, the main difference between both species is that C. gattii affects immunocompetent people and is not associated to HIV infection (Sorrell, 2001). In some regions such as the North of Brazil, C. gatti is endemic and about the 60% of the cryptococcosis cases can be attributed to it (Nishikawa et al., 2003).

The most characteristic clinical symptoms thereof are generalized pneumonia with severe lung damage, with meningitis being detected in a minority of the cases in which there is usually some immunological defect (Harris et al., 2011; Ngamskulrungroj et al., 2012). Pulmonary cryptococcosis is characterized by a feverish condition with dyspnea, dry cough, nighttime perspiration and progressive respiratory deterioration, being occasionally fulminant. Severe pulmonary conditions involve a risk of dissemination to the CNS and other locations, especially the skin. The main risk factor is HIV infection, particularly with CD4 lymphocytes value under 100 cells/mm³, being CNS involvement dominating the clinical symptoms. In other immunosuppression conditions, such as pulmonary chronical disease, patients undergoing long-term corticotherapy or patients receiving a solid organ transplant, a predominant respiratory condition is produced.

In spite of constituting only 1% of all the worldwide cryptococcosis infections, C. gatti acquired significant importance when, in 1999, this species was found to be the agent causing an outbreak detected in Vancouver island, Canada (Hoang et al., 2004). These infective strains have currently extended to other regions in Canada and Northeast Pacific (Byrnes and Marr, 2011; Galanis and Macdougall, 2010). Thus, the study of C. gattii virulence is a subject which arouses great interest within the scientific community.

### Immune response function

It is crucial that the host immune system responds correctly so as to eradicate or limit extension of the fungal pathogens from the lungs, before an extensive dissemination takes place. Thereby, the host innately have phagocytic lung cells as a first defence line. Said cells include macrophages, dendritic cells and neutrophils. When the fungus is recognized by phagocytes, these absorb and neutralize the invading organism forming the phagosome, which will maturate, acidify and then fuse with a lysosome leading to the fungus destruction. However, the Cryptococcus has developed mechanisms and virulence factors to avoid the elimination thereof, and although it has been outlined in previous sections, it is important to underline the complex and elaborated structure of the capsule thereof, formed mainly by glucuronoxylomannan (GMX), galactoxylomannan (GalXM) and mannoproteins anchored to the cell wall, also including chitin, chitosan, glucans and glycoproteins.

The capsule is dynamic and during infection it increases the thickness thereof drastically. Additionally, it prevents Cryptococcus from being recognized as a pathogen-associated molecular pattern (PAMPs), allowing it to avoid the immune system and phagocytosis. Furthermore, it makes protection thereof easier against reactive oxygen species (ROS) and antimicrobial peptides, and it has anti-phagocytic properties involved in opsonization. Another mechanism employed by this fungus is the ability thereof to form "titan cells", as it has been described above (Zaragoza et al., 2010).

Finally, melanin production catalysed by laccase should be highlighted as another virulence factor of both species, since it protects the organism from oxidative stress inside the phagolysosome by breaking and transforming harmful substrates from the host in intermediate reagents which can damage the host itself.

### Diagnosis and isolation

The usual radiological pattern of Cryptococcus is multiple nodular involvement. The reticulonodular patterns are more characteristic of invasive symptoms. Involvement such as lobar or segmental consolidation is also possible. Thoracic adenopathy may also appear. Pleural effusion or cavitation is exceptional.

Its presence as colonizer is not usually frequent, so the isolation thereof in culture of sputum or BAL in the clinical context is enough for diagnosis. Determining the Cryptococcus antigen in plasma is very useful, but it can be negative in the limited pulmonary forms. Detection of the antigen in sputum or BAL shows inconsistent results. Study of the cerebrospinal fluid (CSF) is realized by staining with Chinese ink. Lumbar puncture is not recommended as a routine in all the pulmonary forms, but only in presence of neurological symptoms, severe immunosuppression, dissemination data, or high titres of antigen in serum.

### Treatment

Treatment of the pulmonary forms has not been studied much and the main recommendations are based in the therapeutic experience of CNS involvement in patients undergoing HIV infections. In asymptomatic patients the application of anti-fungal agents is not indicated, so with mild or moderate symptoms fluconazole is recommended for 6-12 months. With severe symptoms or with CNS involvement, it is recommended at least 2 weeks of combined therapy with amphotericin B and fluorocytosine. After this treatment, monitoring with fluconazole is continued for 6-12 months. The length of the treatment must be individualized depending on the risk factors and on whether the immunosuppression state has been reverted. Thus, in subjects suffering from HIV it must be kept until viral replication is controlled and an amount of CD4 (>100 cells/mm³) lymphocytes is stably obtained. Furthermore, the start of the antiretroviral therapy must be delayed at least 4-8 weeks in order to avoid the immune reconstitution syndrome, underlying once more the difficult immunological balance required to manage opportunistic mycosis.

An inconvenience lies in the fact that numerous pathogenic strains of C. neoformans are refractory to the application of echinocandins, acting as specific inhibitors of glucan synthase which is involved in the β (1,3)-glucan synthesis, a main component in the fungal wall. New formulations are being used to try and solve this circumstance.

### Therapy based on natural substances

Although active substances from natural origin extracts such as those from the following plants: *Cuminum cyminum, Salvadora persica, Syngonanthus nitens, Tulbaghia alliacea, Alternaria alternata, Trichoderma spp., Arthrinium arundinis, Selaginella tamariscina, Glycyrrhizine* and *Citrus bergamia,* have been studied in different scientific experiments, none of them have proved to be an efficient antifungal against infections by Cryptococcus (relevant quotes in the section "References").

Regarding research as well as application thereof to the existent pathologies in the subject matter at hand, there are some gaps in terms of application and effectiveness. In this sense, in the current state of the art each pathology and disorder is found to be treated in a sectorial and individual way; so the active substance used is different for trying to fight pyorrhoea, cryptococcosis or decay by *Streptococcus mutans.* The solutions offered are then unilateral and segmented. In the case of cryptococcal meningitis, the few active substances being offered not only do not contemplate global loss of functionality, but also no product is currently patented which can fight it completely, since each single active substance or combinations tested have failed in the noble and laudable purpose of eliminating the pathology.

Designing an effective strategy against the harmful activity of the Cryptococcus species being described, demands a double aspect concept, molecular on the one hand and multi-target on the other. The combinations of natural extracts or products randomly chosen do not lead, in any case, to obtaining results which are positive, appropriate and susceptible of being applicable in the clinical practice.

The lamiaceae are a constant source of natural substances featuring proven therapeutic value. This is a wide and exceptional family of angiosperm dicotyledon plants and shrubs, characterized by comprising a stem with a square cross-section, opposing and decussate leaves, hermaphroditic flowers, frequently zygomorphic, being colourful, with persistent calyx and corolla with fused petals (sympetalae), the end of which finishes into two portions or lips (bilabiatae), the upper one being formed by two petals and the lower one by three. Regarding their fruit, this is dry and formed by four nuculas. Broadly known examples are rosemary, basil, lavender, thyme and sage.

The existing bibliography collects several studies in which compounds form Rosmarinus officinalis (rosemary) is used against cryptococosis, but always using the essential oil moiety containing exclusively monoterpenes: cineole, camphor, borneol, verbenone, etc, and not the polyphenolic moiety containing the diterpenes, and other compounds such as triterpenes and caffeic acid derivatives.

In our research an extract was used highly and specifically enriched in diterpenes (at a concentration higher than 80%) and above all in carnosic acid (at a concentration higher than 70%), with additional presence of little portions of carnosol and other diterpenes with a similar structure. Carnosic acid, the structure of which is shown below, is a phenolic diterpene which is extracted from leaves of the rosemary plant (Rosmarinus officinalis) and the antioxidant, anticancer, neuroprotective and anti-inflammatory properties thereof, as well as the preservative effect thereof for products of diverse nature, are broadly known.

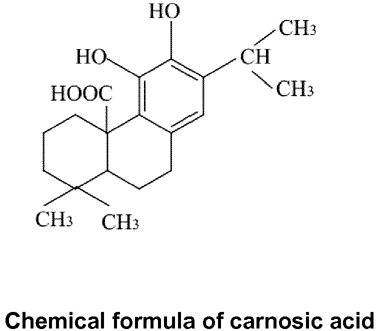

Currently, the bibliographic information about acting mechanisms of this multi-functional compound is that detailed as follows:
- It does not block the histamine release.
- It reduces the release of NO, PGE₂, TNF-alpha induced by PGN.
- It deletes, at a transcription level, the inflammatory response. Src/Syk could be the object of said response, since kinase activity thereof is eliminated.
- It is a powerful chemoprotective agent against oral carcinogenesis, probably due to the antilipoperoxidative potential and modulating effect thereof in carcinogenic detoxification of enzymes during DMBA-induced oral carcinogenesis.

Currently, the use of rosemary extracts rich in diterpenes is focused exclusively in their application as antioxidant agents, capable of preventing oxidation, "Rancidification" of lipids and some proteins, however the use thereof as "antimicrobial" agents is almost none. Regarding propolis, it is well known that it is a natural product produced by bees, as a result of the addition of mandibular secretions to resins collected thereby from different plants. In the beehive they are used for diminishing inlets, sealing fissures and embalming dead organisms. The composition thereof varies considerably depending on the collecting place, the plants used for producing it and on the concrete bee species. Among its properties the antimicrobial, antioxidant and anti-tumour actions should be highlighted.

There are records of a certain amount of scientific studies about the use of propolis from different nature in treating certain fungal infections. However, although said studies suggest that theses extracts have certain antifungal potential, none of them conclusively asserts an effective, reliable and safe application for eradication of the different types of cryptococcosis.

In short, through experimental evidence obtained in the present invention, it is demonstrated that only the adequate combination of both extracts show a reliable efficiency in the potential treatment of cryptococcal meningitis. The results obtained allow reaching the conclusion of a significant molecular synergy between carnosic acid (diterpenes) together with polyphenols and flavonoids present in propolis, even overcoming the usual variability in distribution of polyphenols of the latter.

It should be reiterated that, currently, the number of synthetic/pharmacologic antifungals and natural products intended to eradicate cryptococcosis is increasing, in parallel with the high prevalence of this yeast in nosocomial infections in humans. However, efficiency of these compounds is not able to solve the existing sanitary problem. And, in spite of the efforts of the scientific community, a really effective product has not been achieved yet. In this regard, some families of antifungals should be highlighted that are internationally commercialized, being described depending on the active substance contained therein and the administration route thereof:

### Oral route

- Fluconazole: also commercialized as Fluconazole Apotex, Diflucan and Gynflu-P.
- Mycostatin: also commercialized as Nystatin, Bio-Statin or Mycostatin.
- Itraconazole: also commercialized as Sporanox.
- Amphotericin B: available as Ambisome.
- Ketoconazole: also commercialized as Nizoral.
- Voriconazole: also commercialized as Vfend.
- Nilstat: also commercialized as Infestat, Korostatin, Mycostatin, Mykinac, Nysert, Nystalocal, Nystamont, Nystan, Nystatin, Nystex, Nystop.
- Candifix
- Clarithromycin.
- Terbinafine: also commercialized as Lasimil.
- Micafungin.

### Topic route

- Clotrimazole: also commercialized as Gine canesten, Glynclox Lafrancol and Lotrimil.
- Myconazole: also commercialized as Daktarin and Gynflu-P
- Secnidazole: also commercialized as Gynflu-P.
- Clindamycin: also commercialized as Gynclox Lafrancol.
- Mycostantin: also commercialized as Nystatin, Bio-Statin or Mycostatin.
- Econazole.
- Fenticonazole.
- Ketoconazole: also commercialized as Nizoral.
- Sertaconazole.
- Tioconazole.
- Terbinafine: also commercialized as Lasimil.

### Parenteral route

- Amphotericin B.
- Voriconazole: also commercialized as Vfend.
- Clarithromycin.
- Caspofungin.
- Micafungin.

### Ovules

- Butoconazole: also commercialized as Femstat.
- Clindamicyn: also commercialized as Gynclox Lafrancol.
- Nystatin.
- Ketoconazole: also commercialized as Nizoral.

Regarding potential substances of natural origin, there are anti-cryptococcal compositions in the market with low effectivity and without a scientific basis supporting their foreseeable antifungal effects, since no decisive experimental tests have been carried out. Products of this kind can be found both of probiotic nature (*Saccharomyces boulardii, Lactobacillus acidophilus, etc*.) and vegetal origin (grapefruit seed extract, aloe vera, garlic, etc.), resulting in heterogeneous mixtures without an established synergy, being mere "cocktails" which try to cover action spectra randomly and poorly efficiently, obtaining mainly commercial products.

Since said mixtures are complex and unspecific both in the composition and in the acting mechanisms thereof, some have even included- without any synergistic basis-, among more than fifteen or twenty compounds, the propolis or some extracts from Lamiaceae plants containing polyphenols of the caffeic acid, but not of diterpenic nature (absence of carnosic acid and other diterpenes). Said products are commercialized in naturopathy shops and specific portals for online shopping, without any medical or scientific basis. As an example, it should be mentioned: "Puri-corp", "Holoprolis spray", "Candaway", "Candinorm" or "Candi clear".

Unlike the cases cited, in the present application, the experimental assays realized rigorously and following a standard, show the efficiency of the formulation disclosed, concluding that the magnitude of the resulting synergy significantly exceeds the most effective anti-cryptococcosis products used so far. As well as being supported by clear scientific tests, the present application is not based in an unspecific mixture which leads to a "cocktail" encompassing very wide fields of action, so as to guarantee not only that at least some of the compounds being added are really efficient in the intended application, but also that the two compounds forming the basis of the patent and the concrete application thereof have been specifically selected after a deep analysis.

So far, there has not been found any document from the state of the art which describes the use of active principles being used therein to fight against cryptococcal Meningoencephalitis in the different representations thereof.

### DESCRIPTION OF THE INVENTION

The present application shows a high level of innovation in the chemical composition thereof, as it is shown by the following evidence.
- There is no document in the state of the art, related to the object of the present invention which has described the use of labiatae plants extracts containing diterpenes in proportions greater than 80% and with a concentration of carnosic acid being higher than 70%, combined with propolis extracts containing total polyphenols at a concentration between 70 and 90% by weight.
- In the present invention rosemary essential oil has not been used nor is carnosic acid included in any way so as to avoid oxidation or ageing. Therefore, the present composition is directed to treat cryptococcosis in all its forms and representations, either topic or systemic, testing rigorously, exactly and scientifically the assertions being done herein through cell death of Cryptococcus observed in the kinetics and dose-response assays supporting this application.

The present invention provides a synergistic composition comprising
- Propolis. Including polyphenols at a concentration between 70 and 90% by weight with respect to propolis and
- carnosic acid
For the use thereof in preventing and/or treating infections caused by species of the opportunistic fungus neoformans selected from the group consisting of cryptococcosis, meningitis and pneumonias in humans and/or animals.

The invention is also defined as a method for preventing and/or treating infections caused by species of the opportunistic fungus *Cryptococcus neoformans* selected from the group consisting of cryptococcosis, meningitis and pneumonias in humans and/or in animals, which involves administering a synergistic composition comprising
- propolis comprising polyphenols at a concentration between 70 and 90% by weight with respect to propolis and
- carnosic acid.

The invention is also defined as the use of a synergistic composition comprising:
- propolis containing polyphenols at a concentration between 70 and 90% by weight with respect to propolis and
- carnosic acid.
for elaborating a drug for preventing and/or treating infections caused by species of the opportunistic fungus *Cryptococcus neoformans* selected from the group consisting of cryptococcosis, meningitis and pneumonias in humans and/or in animals.

Another embodiment is a synergistic composition consisting of:
- propolis comprising polyphenols at a concentration between 70 and 90% by weight with respect to propolis and
- carnosic acid,
for the use thereof for preventing and/or treating infections caused by species of the opportunistic fungus *Cryptococcus neoformans* selected from the group consisting of cryptococosis, meningitis and pneumonias in humans and/or in animals.

The synergistic composition of the invention is used, by administrating an effective dose, as a fundamental product for preventing and/or fighting cyptococcosis in the mucosa of the vaginal tract, both as a gel, cream, ointment and ovules, and also as hygienic towels.

The synergistic composition of the invention is used, by administrating an effective dose thereof, as a fundamental product for preventing and/or fighting cryptococcosis in cases in which it appears as a very virulent pathogenic agent, as it happens when the immune system suffers from severe suppression or weakening, in patients undergoing intensive surgery, those receiving a transplant or being monitored in intensive care units, neonates, old people and people undergoing anti-tumour therapy or long-lasting antibiotic treatments; as well as the outbreak of invasive cryptococcosis in people undergoing HIV and septicaemia.

The combination of propolis comprising polyphenols at a concentration between 70 and 90% by weight with respect to propolis and carnosic acid is also used as bioactive ingredient in the preparation of devices and objects for pets, as well as in several veterinarian applications.

Another embodiment is the synergistic composition of the invention, wherein the concentration of the propolis is from 20 and 80% by weight with respect to the total amount of the synergistic composition.

Another embodiment is the synergistic composition of the invention, wherein the concentration of carnosic acid is between 10 and 60% by weight with respect to the synergistic composition total.

Another embodiment is the synergistic composition of the invention, wherein cryptococcosis is of epithelial type.

The synergistic compositions of the invention is used, by administrating an effective dose thereof, as a fundamental product for preventing and/or eradicating epithelial cryptococcosis, as well as in the scalp and nails.

Another embodiment is the synergistic composition of the invention, which is selected from the group comprising cream, gel, ointment, ovules, spray, tablets, topical use powder, capsules, powder for oral suspension, ear drops, toothpaste, mouthwash, perfusion, syrup, towels, dental silk, dental floss, toothbrush and interdental brush.

The synergistic composition of the invention is used as a spray (pulverization) for preventing and/or fighting buccopharyngeal and bronchial conditions.

The synergistic composition of the invention is used as ear drops for preventing and/or treating otitis.

The synergistic composition of the invention is used as a fundamental product for prevention and maintenance of a correct oral hygiene, as well as for dental prosthesis, especially in the case of diabetic users, and dental implants susceptible of developing cryptococosis, through tooth paste, mouthwash and cleaning liquids.

The invention also provides a pharmaceutical and/or veterinarian synergistic composition comprising:
- propolis comprising polyphenols at a concentration between 70 and 90% by weight with respect to propolis and
- carnosic acid,
together with pharmaceutically and/or veterinarially acceptable excipients, for the use thereof for preventing and/or treating infections caused by the opportunistic fungus species Cryptococcus neoformans selected from the group consisting of cryptococosis, meningitis and pneumonia in humans and/or in animals, from now on the pharmaceutical and/or veterinarian composition of the invention.

In the present invention, by "excipient" is meant that matter that, when included in the pharmaceutical forms, is added to the active substances or the associations thereof so as to make its preparation and stability possible. Examples of excipients are binding agents, fillers, disintegrating agents, lubricants, coatings, sweeteners, flavouring agents and colouring agents. More concrete non-limiting examples of acceptable excipients are starches, sugars, sorbitol, xylitol, calcium phosphate, spheroid fats, talc, silica or glycerine among others.

Another embodiment is the pharmaceutical and/or veterinarian synergistic composition of the invention, wherein the propolis concentration is between 20 and 80% by weight with respect to the pharmaceutical or veterinarian synergistic composition total.

Another embodiment is the pharmaceutical and/or veterinarian synergistic composition of the invention, wherein the propolis concentration is between 10 and 60% by weight with respect to the pharmaceutical or veterinarian synergistic composition total.

Another embodiment is the pharmaceutical and/or veterinarian synergistic composition of the invention, wherein said excipients are selected from the group consisting of binders, fillers, disintegrating agents, lubricants, coatings, sweeteners, flavouring agents, colouring agents, sugars, xylitol, calcium phosphate, spheroid fats, talc, polysorbate, propylene glycol, isopropyl alcohol, microcrystalline cellulose, magnesium stearate, lactose, lactose monohydrate, rice starch, maltodextrins, sodium lauryl sulphate, sorbitol, light precipitated calcium carbonate, sodium bicarbonate, sodium silicate solution, sodium saccharine, sodium carboxymethyl cellulose, light mineral oil, purified water, colloidal silica dioxide, sucrose, anhydrous colloidal silica, acacia gum, sodium citrate, anhydrous citric acid, sodium chloride, sodium hydroxide, glycerine, hydroalcoholic of glyceryl polymethacrylate, eudermic surfactants, ethanol and benzalkonium chloride.

The invention also provides a synergistic food product comprising:
- propolis comprising polyphenols at a concentration between 70 and 90% by weight with respect to propolis and
- carnosic acid,
for the use thereof for preventing and/or treating infections caused by species of the opportunistic fungus *Cryptococcus neoformans* selected from the group consisting of cryptococosis, meningitis and pneumonias in humans and/or in animals, from now on synergistic food product of the invention.

The combination of propolis comprising polyphenols at a concentration between 70 and 90% by weight with respect to propolis and carnosic acid is also used as bioactive ingredient in food.

Another embodiment is the synergistic food product of the invention selected from the group consisting of chewing gum, jellies, lollipops and sweets.

Another embodiment is the synergistic food product of the invention, wherein the propolis concentration is between 20 and 80% by weight with respect to the food product total.

Another embodiment is the synergistic food product of the invention, wherein the carnosic acid concentration is between 10 and 60% by weight with respect to the food product total.

### BRIEF DESCRIPTION OF THE FIGURES

Figure 1 shows cell morphology by means of optical microscopy about yeasts of *Cryptococcus neoformans* H99, treated with: propolis (PP) at a concentration of 200 µg/ml, carnosic acid (CA) at a concentration of 50 µg/ml and the combination of both compounds (S). The differential interference contrast technique from "Nomarsky" is applied.
Figure 2 shows cell viability in a liquid medium of the strain *Cryptococcus neoformans* H99 in response to treatment with carnosic acid (50 µg/ml), propolis (200 µg/ml) and a synergistic combination of both, taking as reference a control sample with no treatment.
Figure 3 shows a colony growth test in a solid YPD medium of the strain *Cryptococcus neoformans* H99 in response to treatment with carnosic acid (50 µg/ml), propolis (200 µg/ml) and a synergistic combination of both. A negative control was included. C: Control. PP: propolis at a concentration of 200 µg/ml. CA: carnosic acid. S: a combination of both compounds (S).
Figure 4 shows a representation of the existence of synergism through the mathematical model of representation of the isobolographic analysis.
Figure 5 shows a representation of the existence of synergism through the mathematical model of representation of the isobolographic analysis.

### DESCRIPTION OF THE EMBODIMENTS

### Example 1. Synergistic effect of the combination of propolis and carnosic acid on Cryptococcus neoformans

The strain used in this example is a strain of the yeast *Cryptococcus neoformans* H99. This strain has been treated with: propolis (PP) at a concentration of 200 µg/ml, carnosic acid (CA) at a concentration of 50 µg/ml and a combination of both compounds (S).

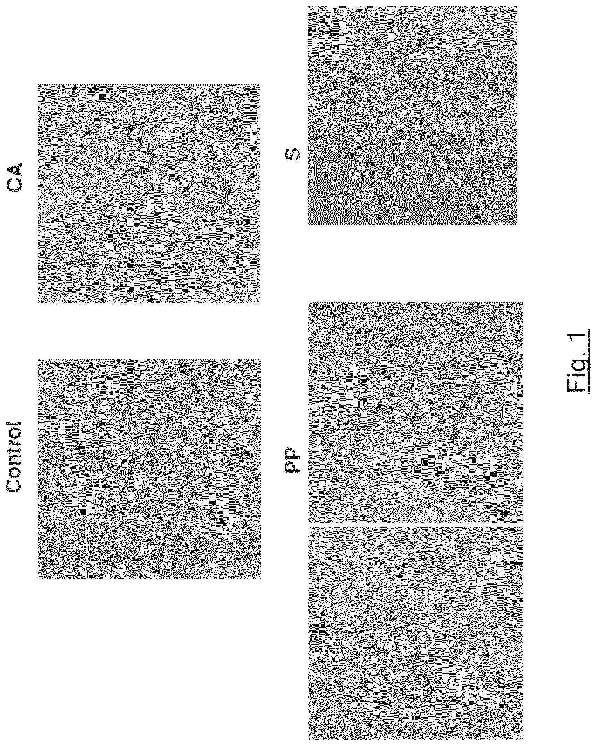

The cell morphology of the strain has been observed by optical microscopy. The differential interference contrast technique from "Nomarsky". Although a slight granularity in the cells treated with the compounds individually, those which have been exposed to the combination of said compounds, have a strongly higher granularity, as well as morphological alterations being visible in the cells, which can be interpreted as structural alterations of the outer structure (wall) with the subsequent cell death. The microscopy optical imaging are shown in Figure 1.

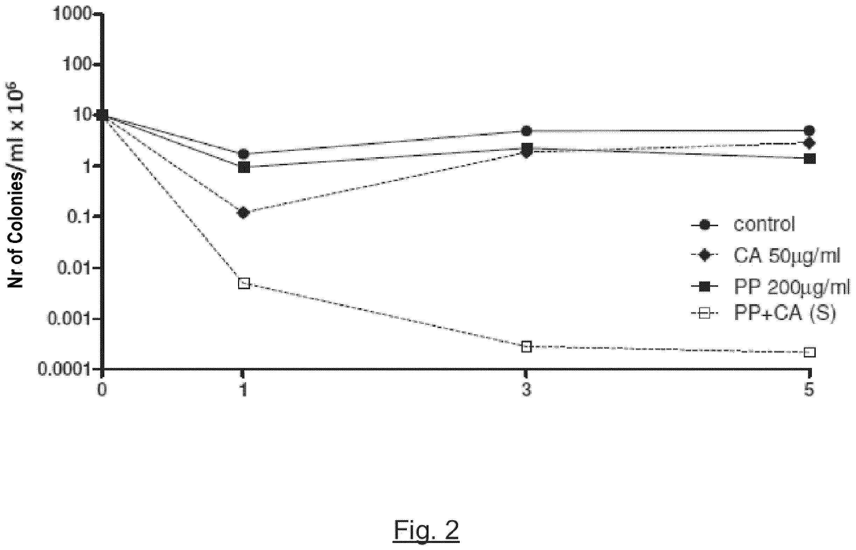

Cell viability measures have been performed in liquid medium of the strain in response to treatment with propolis (PP) at a concentration of 200 µg/ml, carnosic acid (CA) at a concentration of 50 µg/ml and the combination of both compounds (S), taking as a reference a control sample, with no treatment. The assay started from a pre-inoculum incubated overnight at 28°C; the next day it was refreshed in the same YPD medium (D.O. 600nm= 0.3). The culture was allowed to grow until D.O. 600nm= 0.8 at 37°C. Then, the compounds object of the assay were added for one hour at the same temperature, collecting identical aliquots at the indicated times. The results are shown in Figures 1 and 2.

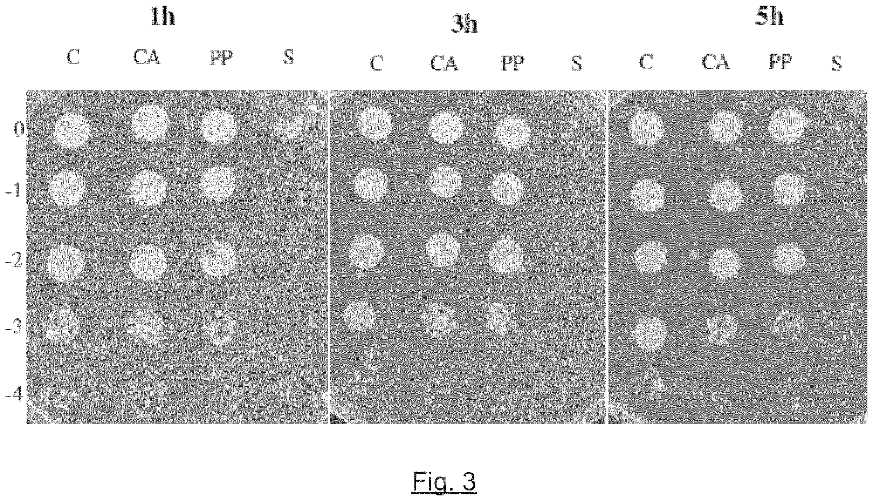

Colony growth assays were performed in solid YPD medium of the strain in response to treatment with propolis (PP) at a concentration of 200 µg/ml, carnosic acid (CA) at a concentration of 50 µg/ml and the combination of both compounds (S), with the purpose of measuring growth inhibition at different exposure times (1, 3 and 5 hours). Additionally, a negative control was included. In order to take the samples, the protocol described in the previous paragraph was followed. Serial dilutions were performed for each sample and 5 µl of each dilution were dispensed on solid medium plates. The plates were incubated for 24-48 hours before being photographed. The results are shown in Figure 3.

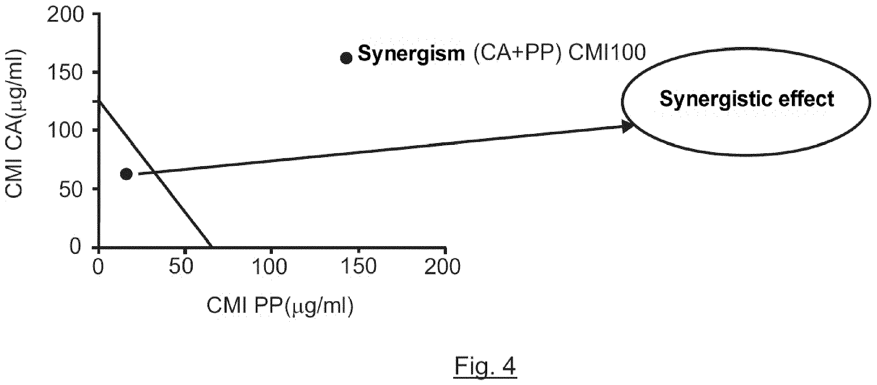

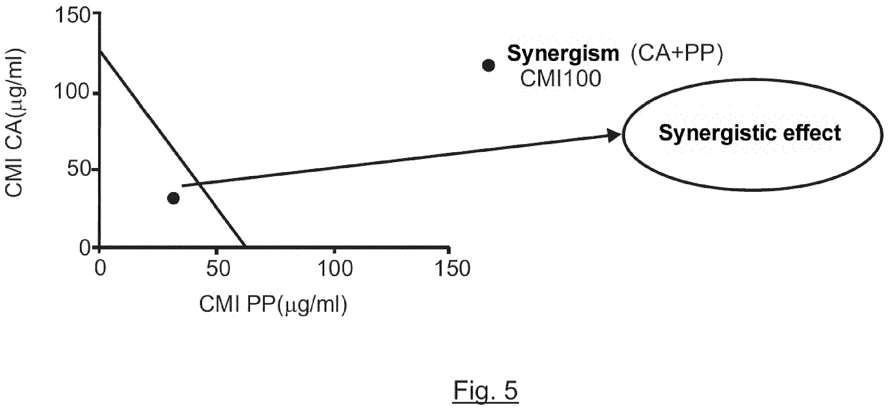

The existence of synergism has been scientifically demonstrated by means of a mathematical model of representation of the isobolographic analysis (Tallarida, RJ, 2012. Quantitative methods for assessing drug synergism. Genes& Cancer, 2: 1003-1008). This reference proposes the use of mathematical formulae (isobolograms) to elucidate if there is synergism or not. The CMI100 (doses) are connected through a line which cuts the axes (Isobolo). If the effect of the compounds combination was additive the mixture of concentrations reducing the population at 100% (CMI100) would fall over the straight line created. Synergism would be underneath said straight line and the antagonism above it. In this case, application of this method to our results shows that the combination of CA (carnosic acid) + PP (propolis) has a strong synergistic effect on strain H99 of *Cryptococcus neoformans.* Isobolograms are shown in Figures 4 and 5.

Microdilution method in M27-S4 plates from CLSI (Clinical Laboratory Sciences Institute). 96-well plates were arranged with serial dilutions of the antifungals, starting from a stock solution in RPMI medium (with glutamine and without sodium bicarbonate), buffered with morpholino propanesulfonic acid (MOPS) 0.164M (ICN, Sigma), adjusted to pH 7.0±0,1, and 0.2% glucose. Subsequently, each well was added 100 µl of the suspension of yeast previously diluted 1000 times in RPMI, until obtaining 1-5x10³ cells/ml. Therefore, the final concentration in the well is of 0.5-2.5x10³ cells/ml. The plates were inoculated at 37°C for 24h, in a wet environment so as to prevent evaporation of the medium. After incubation, a visual reading of the CMIs was performed based on the colony intensity in the well bottom. Each test was duplicated, using the most representative data. In all plates Growth Controls (GC) and Sterility Controls (SC) were taken into account for all concentrations.

**Table 1. Results obtained after 48 hours**

| | **GC*** | **CMI₁₀₀ µg/ml** |
|---|---|---|
| **Carnosic acid (CA)** | **+** | **125** |
| **Propolis (PP)** | **+** | **62.5** |
| **Synergism (CA + PP)** | **+** | **62.5/15.6** |

GC corresponds to Growth Control. For each of the concentrations tested, it has been confirmed that there was growth of the microorganism, with the objective of verifying that the absence of said microorganism was only due to the antifungal capacity of the compounds being administered. Similarly, for each concentration used in said assay, a Sterility Control (SC) was also included, being thus certain that the sterile conditions of the medium are maintained, avoiding thus false positives.

Figura 4 corresponds to the results in Table 1.

**Table 2. Results obtained after 48 hours**

| | **GC*** | **CMI₁₀₀ µg/ml** |
|---|---|---|
| **Carnosic acid (CA)** | **+** | **125** |
| **Propolis (PP)** | **+** | **62.5** |
| **Synergism (CA + PP)** | **+** | **31.25/31.25** |

Figure 5 corresponds to the results in Table 2.

Results in Table 1 (Figure 4) and Table 2 (Figure 5) have been obtained from the same initial stock dissolution. Data are obtained about the synergistic effect for two different dilutions, obtaining thus data of the synergistic effect for two different CMI100 of carnosic acid (CA) and propolis (PP).

### Example 2. Examples of ways for application, vehicles and application-dosification systems

### Applicability

Considering the multiple possibilities and need for the application of this synergistic combination, there are included proposals for different dosage manners and systems thereof.

In case of oral health, paste or dental gel represents a simultaneous multifactorial action mechanism, against degeneration-loss of functionality also of a multifactor origin, encompassing both cryptococcosis and avoidance of decays and gingivitis, as well as saliva and bucodental normalization.

In infections in the female urogenital tract, application would take place through towels, considering that mouth and vagina has the same epithelial composition of lysozymes and mucosa. In turn, in systemic cryptococcosis the application could be in the form of a syrup or injection.

In order to establish the correct applicability of the synergistic composition of the present application, all the necessary tests were performed for the suitable adaptation of said formulation with the excipients described below, with the result of the same one being correct in all the cases included:

### CREAM

1. Propylene glycol
2. Polysorbate

### SPRAY

1. Propylene glycol
2. Isopropyl alcohol

### TABLETS

1. Microcrystalline cellulose
2. Magnesium stearate
3. Lactose

### POWDER FOR TOPIC USE

1. Rice starch
2. Maltodextrins

### CAPSULES

1. Lactose or lactose monohydrate
2. Microcrystalline cellulose
3. Corn starch
4. Magnesium stearate
5. Sodium lauryl sulphate
6. Colloidal silica dioxide

### POWDER FOR ORAL SUSPENSION

1. Sucrose
2. Anhydrous colloidal silica
3. Acacia gum
4. Sodium citrate
5. Anhydrous citric acid

### PERFUSION

1. Sodium chloride
2. Sodium hydroxide for pH adjusting

### SYRUP

1. Glycerine

### TOWELS

1. Hydroalcoholic of glyceryl polymethacrylate
2. Eudermic surfactants
3. Ethanol
4. Propylene glycol
5. Benzalkonium chloride

### REFERENCES

Byrnes, E. J., 3rd and Marr, K. A. (2011). The Outbreak of Cryptococcus gattii in Western North America: Epidemiology and Clinical Issues. Curr Infect Dis Rep 13, 256-261.
Campbell, L. T., Fraser, J. A., Nichols, C. B., Dietrich, F. S., Carter, D. and Heitman, J. (2005). Clinical and environmental isolates of Cryptococcus gattii from Australia that retain sexual fecundity. Eukaryot Cell 4, 1410-1419.
Casadevall, A. and Perfect, J. (1998). Cryptococcus neoformans (Washington DC: ASM). Chen, J., Varma, A., Diaz, M. R., Litvintseva, A. P., Wollenberg, K. K. and Kwon-Chung, K. J. (2008). Cryptococcus neoformans strains and infection in apparently immunocompetent patients, China. Emerg Infect Dis 14, 755-762.
Chottanapund, S., Singhasivanon, P., Kaewkungwal, J., Chamroonswasdi, K. and Manosuthi, W. (2007). Survival time of HIV-infected patients with cryptococcal meningitis. J Med Assoc Thai 90, 2104-2111.
Crowe, S. M., Carlin, J. B., Stewart, K. I., Lucas, C. R. and Hoy, J. F. (1991). Predictive value of CD4 lymphocyte numbers for the development of opportunistic infections and malignancies in HIVinfected persons. J Acquir Immune Defic Syndr 4, 770-776.
Currie, B. P. and Casadevall, A. (1994). Estimation of the prevalence of cryptococcal infection among patients infected with the human immunodeficiency virus in New York City. Clin Infect Dis 19, 1029-1033.
Dromer, F., Casadevall, A., Perfect, J. and Sorrell, T. (2011). Cryptococcus neoformans: Latency and Disease, In Cryptococcus. From human pathogen to model yeast, T. R. K.
Joseph Heitman, Kyung J Kwon-Chung, John R Perferct, and Arturo Casadevall, ed. (Washington: ASM), pp. 431-441.
Fortes, S. T., Lazera, M. S., Nishikawa, M. M., Macedo, R. C. and Wanke, B. (2001). First isolation of Cryptococcus neoformans var. gattii from a native jungle tree in the Brazilian Amazon rainforest. Mycoses 44, 137-140.
Friedman, G. D., Jeffrey Fessel, W., Udaltsova, N. V. and Hurley, L. B. (2005). Cryptococcosis: the 1981-2000 epidemic. Mycoses 48, 122-125.
Galanis, E. and Macdougall, L. (2010). Epidemiology of Cryptococcus gattii, British Columbia, Canada,1999-2007. Emerg Infect Dis 16, 251-257.
Harris, J. R., Lockhart, S. R., Debess, E., Marsden-Haug, N., Goldoft, M., Wohrle, R., Lee, S., Smelser, C., Park, B. and Chiller, T. (2011). Cryptococcus gattii in the United States: clinical aspects of infection with an emerging pathogen. Clin Infect Dis 53, 1188-1195.
Hoang, L. M., Maguire, J. A., Doyle, P., Fyfe, M. and Roscoe, D. L. (2004). Cryptococcus neoformans infections at Vancouver Hospital and Health Sciences Centre (1997-2002): epidemiology, microbiology and histopathology. J Med Microbiol 53, 935-940.
Jongwutiwes, U., Kiertiburanakul, S. and Sungkanuparph, S. (2007). Impact of antiretroviral therapy on the relapse of cryptococcosis and survival of HIV-infected patients with cryptococcal infection. Curr HIV Res 5, 355-360.
Lortholary, O., Poizat, G., Zeller, V., Neuville, S., Boibieux, A., Alvarez, M., Dellamonica, P., Botterel, F., Dromer, F. and Chene, G. (2006). Long-term outcome of AIDS-associated cryptococcosis in the era of combination antiretroviral therapy. Aids 20, 2183-2191.
Mirza, S. A., Phelan, M., Rimland, D., Graviss, E., Hamill, R., Brandt, M. E., Gardner, T., Sattah, M., de Leon, G. P., Baughman, W. and Hajjeh, R. A. (2003). The changing epidemiology of cryptococcosis: an update from population-based active surveillance in 2 large metropolitan areas,1992-2000. Clin Infect Dis 36, 789-794.
Ngamskulrungroj, P., Chang, Y., Sionov, E. and Kwon-Chung, K. J. (2012). The primary target organ of Cryptococcus gattii is different from that of Cryptococcus neoformans in a murine model. MBio 3*.*
Nishikawa, M. M., Lazera, M. S., Barbosa, G. G., Trilles, L., Balassiano, B. R., Macedo, R. C., Bezerra, C. C., Perez, M. A., Cardarelli, P. and Wanke, B. (2003). Serotyping of 467 Cryptococcus neoformans isolates from clinical and environmental sources in Brazil: analysis of host and regional patterns. J Clin Microbiol 41, 73-77.
Park, B. J., Wannemuehler, K. A., Marston, B. J., Govender, N., Pappas, P. G. and Chiller, T. M. (2009). Estimation of the current global burden of cryptococcal meningitis among persons living with HIV/AIDS Aids 23, 525-530.
Sorrell, T. C. (2001). Cryptococcus neoformans variety gattii. Med Mycol 39, 155-168. Velagapudi R, Hsueh YP, Geunes-Boyer S, Wright JR, Heitman J. (2009). Spores as infectious propagules of Cryptococcus neoformans. Infect Immun. 77, 4345-55.
Zaragoza, O., Garcia-Rodas, R., Nosanchuk, J. D., Cuenca-Estrella, M., Rodriguez-Tudela, J. L. and Casadevall, A. (2010). Fungal cell gigantism during mammalian infection. PLoS Pathog 6, e1000945.
Freitas Araújo MG1, Pacífíco M, Vilegas W, Dos Santos LC, Icely PA, Miró MS, Scarpa MV, Bauab TM, Sotomayor CE. Evaluation of Syngonanthus nitens (Bong.) Ruhl. extract as antifungal and in treatment of vulvovaginalcandidiasis. Med Mycol. 2013 Oct; 51(7):673-82.
Hwang IS1, Lee J, Jin HG, Woo ER, Lee DG. Amentoflavone stimulates mitochondrial dysfunction and induces apoptotic cell death in Candida albicans. Mycopathologia. 2012 Apr; 173(4):207-18. Epub 2011 Dec 31.
Zhongguo Yi Xue Ke Xue Yuan Xue Bao. Protective effect of glycyrrhizine in mice with systemic Candida albicans infection and its mechanism. 1991 Oct;13(5):380-3.
Naeini A, Naderi NJ, Shokri H. Analysis and in vitro anti-Candida antifungal activity of Cuminum cyminum and Salvadora persica herbs extracts against pathogenic Candida strains. J Mycol Med. 2014 Mar;24(1):13-8. Epub 2013 Nov 8.
Phaopongthai J, Wiyakrutta S, Meksuriyen D, Sriubolmas N, Suwanborirux K. Azole-synergistic anti-candidal activity of altenusin, a biphenyl metabolite of the endophytic fungus Alternaria alternata isolated from Terminalia chebula Retz. J Microbiol. 2013 Dec; 51(6):821-8. Epub 2013 Dec 19.
Vicente MF, Cabello A, Platas G, Basilio A, Díez MT, Dreikorn S, Giacobbe RA, Onishi JC, Meinz M, Kurtz MB, Rosenbach M, Thompson J, Abruzzo G, Flattery A, Kong. L, Tsipouras A, Wilson KE, Peláez F. Antimicrobial activity of ergokonin A from Trichoderma longibrachiatum. J Appl Microbiol. 2001 Nov;91(5):806-13.
Thamburan S, Klaasen J, Mabusela WT, Cannon JF, Folk W, Johnson Q. Tulbaghia alliacea phytotherapy: a potential anti-infective remedy for candidiasis. Phytother. Res. 2006 Oct; 20(10):844-50.
Tallarida, RJ:,2012. Quantitative methods for assessing drug synergism. Genes& Cancer, 2: 1003-1008.

## Claims

1. Synergistic composition comprising
- propolis comprising polyphenols at a concentration between 70 and 90% by weight with respect to propolis and
- carnosic acid,
for use in preventing and/or treating infections caused by species of the opportunistic fungus *Cryptococcus neoformans* selected from the group consisting of cryptococcosis, meningitis and pneumonia in humans and/or in animals.

2. Synergistic composition for use according to claim 1, **characterized in that** the cryptococcosis is epithelial cryptococcosis.

3. Synergistic composition for use according to claim 1 or 2, **characterized in that** the propolis concentration is between 20 and 80% by weight with respect to the synergistic composition total.

4. Synergistic composition for use according to any of claims 1 to 3, **characterized in that** the concentration of carnosic acid is between 10 and 60% by weight with respect to the synergistic composition total.

5. Synergistic composition for use according to any of claims 1 to 4, **characterized in that** it is selected from the group consisting of cream, gel, ointment, ovules, spray, tablets, powder for topic use, capsules, powder for oral suspension, ear drops, toothpaste, mouthwash, perfusion, syrup, towels, dental silk, dental floss, toothbrush and interdental brush.

6. Pharmaceutical and/or veterinarian synergistic composition comprising:
- propolis comprising polyphenols at a concentration between 70 and 90% by weight with respect to propolis and
- carnosic acid,
together with excipients pharmaceutically and/or veterinarially acceptable, for use in prevention and/or treatment of infections caused by species of the opportunistic fungus *Cryptococcus neoformans* selected from the group consisting of cryptococcosis, meningitis and pneumonia in humans and/or in animals.

7. Pharmaceutical and/or veterinarian synergistic composition for use according claim 6, **characterized in that** the propolis concentration is between 20 and 80% by weight with respect to the pharmaceutical and/or veterinarian synergistic composition total.

8. Pharmaceutical and/or veterinarian synergistic composition for use according to claim 6 or 7, **characterized in that** the carnosic acid concentration is between 10 and 60% by weight with respect to the pharmaceutical and/or veterinarian synergistic composition total.

9. Pharmaceutical and/or veterinarian synergistic composition for use according to claims 6 to 8, **characterized in that** said excipients are selected from the group consisting of binders, fillers, disintegrating agents, lubricants, coatings, sweeteners, colouring agents, sugars, xylitol, calcium phosphate, spheroid fats, talc, polysorbate, propylene glycol, isopropyl alcohol, microcrystalline cellulose, magnesium stearate, lactose, lactose monohydrate, rice starch, maltodextrins, sodium lauryl sulphate, sorbitol, light precipitated calcium carbonate, sodium bicarbonate, sodium silicate solution, sodium saccharine, sodium carboxymethyl cellulose, light mineral oil, purified water, colloidal silica dioxide, sucrose, anhydrous colloidal silica, acacia gum, sodium citrate, anhydrous citric acid, sodium chloride, sodium hydroxide, glycerine, hydroalcoholic of glyceryl polymethacrylate, eudermic surfactants, ethanol and benzalkonium chloride.

10. Synergistic food product comprising
- propolis comprising polyphenols at a concentration between 70 and 90% by weight with respect to propolis and
- carnosic acid,
for use in prevention and/or treatment of infections caused by species of the opportunistic fungus *Cryptococcus neoformans* selected from the group consisting of cryptococcosis, meningitis and pneumonia in humans and/or in animals.

11. Synergistic food product for use according to claim 10, **characterized in that** it is selected from the group consisting of chewing gum, jelly, lollypop and sweets.

12. Synergistic food product for use according claim 10 or 11, **characterized in that** the propolis concentration is between 20 and 80% by weight with respect to the food product total.

13. Synergistic food product for use according to any of claims 10 to 12, **characterized in that** the carnosic acid concentration is between 10 and 60% by weight with respect to the food product total.

## Patentansprüche

1. Synergistische Zusammensetzung, umfassend:
- Propolis, die Polyphenole in einer Konzentration von 70 bis 90 Gew.-% in Bezug auf Propolis umfasst, und
- Carnosolsäure,
zur Verwendung bei der Vorbeugung und/oder Behandlung von Infektionen, die durch Spezies des opportunistischen Pilzes *Cryptococcus neoformans* verursacht werden und die ausgewählt sind aus der Gruppe bestehend aus Kryptokokkose, Meningitis und Pneumonie, bei Menschen und/oder Tieren.

2. Synergistische Zusammensetzung zur Verwendung nach Anspruch 1, **dadurch gekennzeichnet, dass** die Kryptokokkose eine epitheliale Kryptokokkose ist.

3. Synergistische Zusammensetzung zur Verwendung nach Anspruch 1 oder 2, **dadurch gekennzeichnet, dass** die Propolis-Konzentration zwischen 20 und 80 Gew.-% in Bezug auf die gesamte synergistische Zusammensetzung beträgt.

4. Synergistische Zusammensetzung zur Verwendung nach einem der Ansprüche 1 bis 3, **dadurch gekennzeichnet, dass** die Konzentration der Carnosolsäure zwischen 10 und 60 Gew.-% in Bezug auf die gesamte synergistische Zusammensetzung beträgt.

5. Synergistische Zusammensetzung zur Verwendung nach einem der Ansprüche 1 bis 4, **dadurch gekennzeichnet, dass** sie aus der Gruppe bestehend aus Creme, Gel, Salbe, Vaginalzäpfchen, Spray, Tabletten, Pulver zur topischen Verwendung, Kapseln, Pulver zur oralen Suspension, Ohrentropfen, Zahnpasta, Mundwasser, Perfusion, Sirup, Tücher, Zahnseide, Zahnfäden, Zahnbürste und Interdentalbürste ausgewählt ist.

6. Pharmazeutische und/oder veterinäre synergistische Zusammensetzung, umfassend:
- Propolis, die Polyphenole in einer Konzentration von 70 bis 90 Gew.-% in Bezug auf Propolis umfasst, und
- Carnosolsäure,
zusammen mit pharmazeutisch und/oder veterinärisch annehmbaren Arzneiträgern, zur Verwendung bei der Vorbeugung und/oder Behandlung von Infektionen, die durch Spezies des opportunistischen Pilzes *Cryptococcus neoformans* verursacht werden und die ausgewählt sind aus der Gruppe bestehend aus Kryptokokkose, Meningitis und Pneumonie, bei Menschen und/oder Tieren.

7. Pharmazeutische und/oder veterinäre synergistische Zusammensetzung zur Verwendung nach Anspruch 6, **dadurch gekennzeichnet, dass** die Propolis-Konzentration zwischen 20 und 80 Gew.-% in Bezug auf die gesamte pharmazeutische und/oder veterinäre synergistische Zusammensetzung beträgt.

8. Pharmazeutische und/oder veterinäre synergistische Zusammensetzung zur Verwendung nach Anspruch 6 oder 7, **dadurch gekennzeichnet, dass** die Konzentration der Carnosolsäure zwischen 10 und 60 Gew.-% in Bezug auf die gesamte pharmazeutische und/oder veterinäre synergistische Zusammensetzung beträgt.

9. Pharmazeutische und/oder veterinäre synergistische Zusammensetzung zur Verwendung nach den Ansprüchen 6 bis 8, **dadurch gekennzeichnet, dass** die Arzneiträger ausgewählt sind aus der Gruppe bestehend aus Bindemitteln, Füllstoffen, Sprengmitteln, Gleitmitteln, Beschichtungen, Süßstoffen, Farbstoffen, Zuckern, Xylitol, Calciumphosphat, Kugelfetten, Talkum, Polysorbat, Propylenglykol, Isopropylalkohol, mikrokristalliner Cellulose, Magnesiumstearat, Laktose, Laktosemonohydrat, Reisstärke, Maltodextrinen, Natriumlaurylsulfat, Sorbitol, leichtem gefälltem Calciumcarbonat, Natriumbicarbonat, Natriumsilikat-Lösung, Saccharin-Natrium, Natriumcarboxymethylcellulose, leichtem Mineralöl, gereinigtem Wasser, kolloidaler Kiselerde, Saccharose, wasserfreier kolloidaler Kieselerde, Gummi arabicum, Natriumcitrat, wasserfreier Zitronensäure, Natriumchlorid, Natriumhydroxid, Glycerin, wässrig-alkoholischen Lösungen von Glycerylpolymethacrylat, hautfreundlichen Tensiden, Ethanol und Benzalkoniumchlorid.

10. Synergistisches Lebensmittelprodukt, umfassend:
- Propolis, die Polyphenole in einer Konzentration von 70 bis 90 Gew.-% in Bezug auf Propolis umfasst, und
- Carnosolsäure,
zur Verwendung bei der Vorbeugung und/oder Behandlung von Infektionen, die durch Spezies des opportunistischen Pilzes *Cryptococcus neoformans* verursacht werden und die ausgewählt sind aus der Gruppe bestehend aus Kryptokokkose, Meningitis und Pneumonie, bei Menschen und/oder Tieren.

11. Synergistisches Lebensmittelprodukt zur Verwendung nach Anspruch 10, **dadurch gekennzeichnet, dass** es aus der Gruppe bestehend aus Kaugummi, Gelee, Lutscher und Süßigkeiten ausgewählt ist.

12. Synergistisches Lebensmittelprodukt zur Verwendung nach Anspruch 10 oder 11, **dadurch gekennzeichnet, dass** die Propolis-Konzentration zwischen 20 und 80 Gew.-% in Bezug auf das gesamte Lebensmittelprodukt beträgt.

13. Synergistisches Lebensmittelprodukt zur Verwendung nach den Ansprüchen 10 bis 12, **dadurch gekennzeichnet, dass** die Konzentration der Carnosolsäure zwischen 10 und 60 Gew.-% in Bezug auf das gesamte Lebensmittelprodukt beträgt.

## Revendications

1. Composition synergique comprenant
- de la propolis comprenant des polyphénols à une concentration comprise entre 70 et 90% en poids par rapport à la propolis et
- de l'acide carnosique,
à utiliser dans la prévention et/ou le traitement d'infections provoquées par l'espèce du mycète opportuniste *Cryptococcus neoformans* sélectionnées dans le groupe composé de la cryptococcose, méningite et pneumonie chez les humains et/ou chez les animaux.

2. Composition synergique à utiliser selon la revendication 1, **caractérisée en ce que** la cryptococcose est la cryptococcose épithéliale.

3. Composition synergique à utiliser selon la revendication 1 ou 2, **caractérisée en ce que** la concentration de propolis est comprise entre 20 et 80% en poids par rapport à la composition synergique totale.

4. Composition synergique à utiliser selon l'une quelconque des revendications 1 à 3, **caractérisée en ce que** la concentration d'acide carnosique est comprise entre 10 et 60% en poids par rapport à la composition synergique totale.

5. Composition synergique à utiliser selon l'une quelconque des revendications 1 à 4, **caractérisée en ce qu'**elle est sélectionnée dans le groupe composé de crème, gel, pommade, ovules, spray, comprimés, poudre à usage topique, gélules, poudre pour suspension orale, gouttes otiques, pâte dentifrice, bain de bouche, perfusion, sirop, serviettes, soie dentaire, fil dentaire, brosse à dents et brossette interdentaire.

6. Composition synergique pharmaceutique et/ou vétérinaire comprenant :
- de la propolis comprenant des polyphénols à une concentration comprise entre 70 et 90% en poids par rapport à la propolis et
- de l'acide carnosique,
conjointement avec des excipients de qualité pharmaceutique et/ou vétérinaire, à utiliser dans la prévention et/ou le traitement d'infections provoquées par l'espèce du mycète opportuniste *Cryptococcus neoformans* sélectionnées dans le groupe composé de la cryptococcose, méningite et pneumonie chez les humains et/ou chez les animaux.

7. Composition synergique pharmaceutique et/ou vétérinaire à utiliser selon la revendication 6, **caractérisée en ce que** la concentration de propolis est comprise entre 20 et 80% en poids par rapport à la composition synergique pharmaceutique et/ou vétérinaire totale.

8. Composition synergique pharmaceutique et/ou vétérinaire à utiliser selon la revendication 6 ou 7, **caractérisée en ce que** la concentration d'acide carnosique est comprise entre 10 et 60% en poids par rapport à la composition synergique pharmaceutique et/ou vétérinaire totale.

9. Composition synergique pharmaceutique et/ou vétérinaire à utiliser selon les revendications 6 à 8, **caractérisée en ce que** lesdits excipients sont sélectionnés dans le groupe composé de liants, agents de remplissage, agents de désintégration, lubrifiants, enrobages, édulcorants, colorants, sucres, xylitol, phosphate de calcium, graisses sphéroïdes, talc, polysorbate, propylène glycol, alcool isopropylique, cellulose microcristalline, stéarate de magnésium, lactose, lactose monohydraté, amidon de riz, maltodextrines, laurylsulfate de sodium, sorbitol, carbonate de calcium précipité léger, bicarbonate de sodium, solution de silicate de sodium, saccharine de sodium, carboxyméthylcellulose de sodium, huile minérale légère, eau purifiée, dioxyde de silice colloïdale, saccharose, silice colloïdale anhydre, gomme d'acacia, citrate de sodium, acide citrique anhydre, chlorure de sodium, hydroxyde de sodium, glycérine, hydro-alcoolique de polyméthacrylate de glycéryle, surfactants eudermiques, éthanol et chlorure de benzalkonium.

10. Produit alimentaire synergique comprenant
- de la propolis comprenant des polyphénols à une concentration comprise entre 70 et 90% en poids par rapport à la propolis et
- de l'acide carnosique,
à utiliser dans la prévention et/ou le traitement d'infections provoquées par l'espèce du mycète opportuniste *Cryptococcus neoformans* sélectionnées dans le groupe composé de la cryptococcose, méningite et pneumonie chez les humains et/ou chez les animaux.

11. Produit alimentaire synergique à utiliser selon la revendication 10, **caractérisé en ce qu'**il est sélectionné dans le groupe composé de chewing-gum, gelée, sucette et bonbons.

12. Produit alimentaire synergique à utiliser selon la revendication 10 ou 11, **caractérisé en ce que** la concentration de propolis est comprise entre 20 et 80% en poids par rapport à la totalité du produit alimentaire.

13. Produit alimentaire synergique à utiliser selon l'une quelconque des revendications 10 à 12, **caractérisé en ce que** la concentration d'acide carnosique est comprise entre 10 et 60% en poids par rapport à la totalité du produit alimentaire.
